# FASCICULE DE BREVET EUROPEEN

(11) **EP 2 999 426 B1**
(45) Date de publication et mention de la délivrance du brevet: **03.05.2017**
(21) Numéro de dépôt: 14726938.5
(22) Date de dépôt: 21.05.2014
(51) Int. Cl.: A61F 2/00, A61F 5/00, A61B 5/00

(54) **SYSTEME OCCLUSIF IMPLANTABLE COMPRENANT UN DISPOSITIF DE DETECTION DE L'ATROPHIE D'UN CONDUIT NATUREL**
IMPLANTIERBARES VERSCHLUSSSYSTEM MIT EINER VORRICHTUNG ZUR DETEKTION VON ATROPHIE EINER NATÜRLICHEN LEITUNG
IMPLANTABLE OCCLUSION SYSTEM COMPRISING A DEVICE FOR DETECTING ATROPHY OF A NATURAL CONDUIT

(30) Priorité: 21.05.2013 FR 1354534
(43) Date de publication de la demande: 30.03.2016
(73) Titulaire: Uromems, 38040 Grenoble (FR)
(72) Inventeur: LAMRAOUI, Hamid, F-38000 Grenoble (FR)
(74) Mandataire: Regimbeau
(86) Numéro de dépôt international: PCT/EP2014/060467
(87) Numéro de publication internationale: WO 2014/187870

(56) Documents cités:
- US-A1- 2007 156 013
- US-A1- 2011 208 220
- US-A1- 2012 184 980

## Description

### DOMAINE DE L'INVENTION

La présente invention concerne un système occlusif implantable comprenant un élément occlusif et un dispositif pour détecter l'atrophie d'un conduit naturel du corps humain ou animal entouré dudit élément occlusif.

Cette invention s'applique à tout type de système d'occlusion, incluant les sphincters artificiels urinaire, anal, oesophagien ou pylorique ou encore les anneaux gastriques.

### ARRIERE PLAN DE L'INVENTION

L'implantation de systèmes occlusifs pour occlure totalement ou partiellement un conduit naturel d'un patient est connue pour différentes indications.

Par exemple, le traitement de l'incontinence urinaire peut impliquer l'implantation, chez un patient, d'un sphincter artificiel.

Un tel sphincter comprend typiquement un élément occlusif placé autour de l'urètre (chez l'homme ou la femme), parfois du col vésical (chez la femme) ou de la prostate (chez l'homme) dans le but d'exercer une compression directe ou indirecte sur l'urètre afin d'éviter des fuites urinaires, un dispositif d'actionnement dudit élément occlusif pour faire varier la compression exercée sur l'urètre ou le col vésical, ainsi qu'une unité de commande du dispositif d'actionnement.

Un tel sphincter artificiel est décrit en particulier dans [1].

Différentes technologies de sphincters artificiels ont été proposées, basées notamment sur différents types d'éléments occlusifs et de mode d'actionnement associé.

Selon une forme d'exécution, l'élément occlusif est une manchette gonflable contenant un volume variable d'un fluide.

Le dispositif d'actionnement est un dispositif hydraulique, comprenant un réservoir dudit fluide et un actionneur permettant d'ajouter ou de retirer ledit fluide pour compresser ou décompresser la manchette.

Un tel sphincter artificiel est décrit par exemple dans [2].

Un autre exemple de sphincter artificiel est décrit dans [3].

Il existe également un sphincter électromécanique, dans lequel l'élément occlusif est une bande entourant l'urètre ou le col vésical et reliée à un câble qui permet d'exercer une tension plus ou moins forte sur la bande [4].

Les documents US 2007/0156013 et US 2011/0208220 décrivent des bandes gastriques comprenant un capteur permettant de mesurer la pression exercée par l'élément occlusif sur le conduit naturel.

En raison de la compression exercée par l'élément occlusif, la région située sous ledit élément est peu vascularisée, de sorte que la nutrition des tissus est localement réduite.

Ceci se traduit par un amincissement localisé, ou atrophie, des tissus comprimés.

Cette atrophie est réversible si elle est détectée suffisamment tôt ; ainsi, si l'on relâche la compression de la manchette pendant quelque temps, les tissus se régénèrent et se ré-épaississent.

L'atrophie du conduit est la cause d'une diminution de l'efficacité du système occlusif au cours du temps puisque, le diamètre du conduit devenant plus faible, l'élément occlusif n'est plus apte à procurer l'occlusion souhaitée.

Pour y remédier, il est proposé soit de remplacer l'élément occlusif implanté par un autre de diamètre inférieur, soit d'augmenter la pression de compression pour compenser la diminution du diamètre du conduit [4], [5], [6].

Cependant, ces deux solutions requièrent une nouvelle intervention chirurgicale avec les risques qu'elle implique (infection, etc.) et/ou causent en outre un risque de lésion ou d'érosion du conduit plus élevé.

Une lésion et/ou une érosion des tissus - c'est-à-dire une rupture localisée de la paroi du conduit - peuvent se produire lorsqu'un degré important d'atrophie a été atteint.

Des lésions des tissus non traitées ou une érosion des tissus peuvent augmenter le risque d'infection, dans la mesure où les bactéries présentes dans l'urètre peuvent coloniser les tissus lésés.

Pour traiter une lésion et/ou une érosion, il est nécessaire d'effectuer une nouvelle intervention chirurgicale pour retirer le système implanté.

Une lésion ou une érosion des tissus constitue donc un cas de complication sérieuse de l'implantation du système occlusif.

L'érosion n'est pas un phénomène isolé puisqu'une méta-analyse sur 2606 sphincters urinaires artificiels [5] a montré que 12% des patients implantés d'un sphincter urinaire artificiel souffrent d'une érosion.

Il serait donc souhaitable de pouvoir détecter une atrophie le plus tôt possible afin de pouvoir prévenir une lésion ou une érosion du conduit.

Une mesure directe d'une éventuelle atrophie, qui consisterait à mesurer le diamètre de la partie du conduit entouré par l'élément occlusif, n'est pas envisageable car elle serait très complexe à mettre en oeuvre dans un système implanté.

A l'heure actuelle, aucun des systèmes occlusifs proposés sur le marché ne permet de détecter une éventuelle atrophie.

Ce n'est donc que lorsque le patient constate un dysfonctionnement du système occlusif - notamment avec l'apparition de fuites - que le praticien peut considérer que le conduit s'est atrophié.

Un but de la présente invention est donc de concevoir un système occlusif implantable permettant de détecter une éventuelle atrophie de manière fiable et la plus précoce possible et d'alerter en conséquence le praticien ou le patient.

### BREVE DESCRIPTION DE L'INVENTION

Conformément à l'invention, il est proposé un système occlusif implantable dans le corps humain ou animal pour occlure un conduit naturel du corps humain ou animal, comprenant :
- un élément occlusif destiné à entourer une partie du conduit naturel à occlure,
- un dispositif d'actionnement dudit élément occlusif pour faire varier la compression exercée par ledit élément occlusif sur ledit conduit,
- une unité de commande adaptée pour solliciter le dispositif d'actionnement de sorte à exercer une compression déterminée sur le conduit, et
- un dispositif de détection de l'atrophie dudit conduit naturel, comprenant :
   (i) un capteur adapté pour mesurer la compression exercée par l'élément occlusif sur le conduit naturel,
   (ii) une unité de traitement adaptée pour :
      - déterminer un paramètre représentatif d'une sollicitation à appliquer au dispositif d'actionnement pour obtenir une compression déterminée du conduit par l'élément occlusif,
      - surveiller ledit paramètre au cours du temps, et
      - détecter une atrophie du conduit naturel lorsque ledit paramètre représentatif remplit un critère prédéterminé.

De manière particulièrement avantageuse, ledit critère prédéterminé est choisi parmi l'une des conditions suivantes ou une combinaison desdites conditions :
- le paramètre représentatif est supérieur à une valeur fixe,
- le paramètre représentatif est supérieur à un pourcentage d'une valeur dudit paramètre représentatif mesurée initialement,
- une valeur issue d'une fonction mathématique construite à partir d'une base de données de valeurs dudit paramètre représentatif mesurées périodiquement au cours du temps est supérieure à une valeur déterminée.

Selon un mode de réalisation, ledit système est un système occlusif hydraulique dans lequel :
- l'élément occlusif est une manchette gonflable comprenant un volume variable d'un fluide,
- le dispositif d'actionnement comprend un réservoir contenant un fluide, en liaison fluidique avec ladite manchette gonflable,
la variation de la compression exercée par la manchette sur le conduit étant réalisée par transfert d'un volume ajustable dudit fluide entre le réservoir et la manchette.

Selon une forme d'exécution, le capteur est adapté pour mesurer la compression du conduit naturel directement entre l'élément occlusif et le conduit naturel.

Selon une autre forme d'exécution, le capteur est adapté pour mesurer la pression dans une région du circuit hydraulique et l'unité de traitement est configurée pour déduire de ladite mesure la compression exercée par la manchette sur le conduit naturel.

De manière particulièrement avantageuse, le paramètre représentatif surveillé est le volume de fluide à transférer du réservoir dans la manchette gonflable pour atteindre une compression du conduit déterminée.

L'unité de traitement comprend avantageusement une mémoire dans laquelle est enregistrée une relation entre la pression dans le circuit hydraulique et le volume transféré dans la manchette gonflable.

Selon un mode de réalisation, le réservoir est un réservoir à volume variable comprenant une paroi mobile et l'unité de traitement est configurée pour déterminer le volume de fluide transféré à partir d'une mesure du déplacement de ladite paroi mobile.

De manière avantageuse, le système comprend en outre des moyens de détection d'une fuite lente dans le circuit hydraulique, lesdits moyens étant configurés pour :
- la mesure de l'évolution de la pression dans le circuit hydraulique pour une sollicitation déterminée du dispositif d'actionnement,
- la détection d'une fuite lente dans le circuit hydraulique lorsque la pression mesurée dans ledit circuit pour ladite sollicitation déterminée du dispositif d'actionnement remplit un critère prédéterminé.

Ledit critère prédéterminé de détection d'une fuite lente est choisi de préférence parmi l'une des conditions suivantes ou une combinaison desdites conditions :
- la pression dans le circuit hydraulique est inférieure à une valeur fixe,
- la pression dans le circuit hydraulique est inférieure à un pourcentage d'une valeur de la pression mesurée initialement pour ladite sollicitation déterminée du dispositif d'actionnement, et
- une valeur issue d'une fonction mathématique construite à partir d'une base de données de valeurs de ladite pression pour ladite sollicitation du dispositif d'actionnement, ladite pression étant enregistrée périodiquement au cours du temps, est inférieure à une valeur déterminée.

Selon un autre mode de réalisation, le système occlusif est un système occlusif mécanique dans lequel :
- l'élément occlusif est une bande de longueur variable entourant le conduit naturel,
- le dispositif d'actionnement comprend un élément de transmission mécanique reliant ladite bande et un actionneur adapté pour ajuster la course dudit élément de transmission mécanique,
la variation de la compression exercée par la bande sur le conduit étant réalisée par l'ajustement de la course dudit élément de transmission mécanique.

Dans ce cas, le capteur est avantageusement adapté pour mesurer la tension mécanique dudit élément de transmission mécanique et l'unité de traitement est configurée pour déduire de ladite tension mécanique mesurée la compression exercée par la bande sur le conduit naturel.

De préférence, ledit paramètre représentatif est la course dudit élément de transmission mécanique entre l'actionneur et la bande occlusive pour atteindre une compression du conduit déterminée.

L'unité de traitement comprend avantageusement une mémoire dans laquelle est enregistrée une relation entre la course de l'élément de transmission mécanique et la tension mécanique dudit élément de transmission ou la compression exercée par la bande.

De préférence, le paramètre représentatif est défini pour une condition déterminée du patient, le système occlusif comprenant un capteur adapté pour détecter ladite condition déterminée du patient.

Selon un mode de réalisation, l'unité de traitement est incluse dans l'unité de commande du système occlusif.

De manière avantageuse, le système occlusif comprend en outre des moyens d'émission d'une alarme à un utilisateur si le critère de détection d'une atrophie du conduit naturel est rempli.

Selon une application particulière de l'invention, ledit système occlusif consiste en un sphincter urinaire artificiel.

Ledit système permet la mise en oeuvre d'un procédé de détection de l'atrophie d'un conduit naturel entouré par un tel système occlusif, ledit procédé comprenant :
- la mesure - directe ou indirecte - de la compression exercée par l'élément occlusif sur le conduit naturel,
- la détermination d'un paramètre représentatif d'une sollicitation à appliquer au dispositif d'actionnement pour obtenir une compression déterminée du conduit par l'élément occlusif, et la surveillance dudit paramètre au cours du temps,
- la détection d'une atrophie du conduit naturel lorsque ledit paramètre représentatif remplit un critère prédéterminé.

Par exemple, ledit critère prédéterminé est choisi parmi l'une des conditions suivantes ou une combinaison desdites conditions :
- le paramètre représentatif est supérieur à une valeur fixe,
- le paramètre représentatif est supérieur à un pourcentage d'une valeur dudit paramètre représentatif mesurée initialement
- une valeur issue d'une fonction mathématique construite à partir d'une base de données de valeurs dudit paramètre représentatif mesurées périodiquement au cours du temps est supérieure à une valeur déterminée.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma de principe d'un système occlusif hydraulique à associé un dispositif de détection de l'atrophie selon un mode de réalisation de l'invention,
- la figure 2 est un schéma de principe d'un système occlusif hydraulique associé à un dispositif de détection de l'atrophie selon un autre mode de réalisation de l'invention,
- la figure 3 est un schéma de principe d'un système occlusif mécanique associé à un dispositif de détection de l'atrophie selon un autre mode de réalisation de l'invention,
- la figure 4 est un schéma de principe de l'architecture de l'unité de commande d'un système occlusif incorporant un dispositif de détection de l'atrophie,
- la figure 5A illustre un exemple de courbe de variation du paramètre représentatif de la sollicitation à appliquer au dispositif d'actionnement pour obtenir une compression déterminée du conduit par l'élément occlusif en fonction du temps ; la figure 5B présente les différentes positions du piston dans le cas d'un système occlusif hydraulique,
- la figure 6 illustre un exemple de courbe de variation de la pression dans le circuit hydraulique en fonction du temps dans le cas d'une fuite lente.

### DESCRIPTION DETAILLEE DE L'INVENTION

D'une manière générale, le système occlusif comprend un élément occlusif entourant un conduit naturel à occlure.

Selon l'application du système occlusif considéré, le conduit à occlure peut être un conduit urinaire (notamment l'urètre ou le col vésical), anal, oesophagien, pylorique ou encore l'estomac (cas d'un anneau gastrique).

L'occlusion dudit conduit peut être totale (cas d'un sphincter urinaire destiné à éviter des fuites urinaires) ou partielle (cas d'un anneau gastrique destiné à limiter le passage des aliments dans l'estomac).

Le sphincter artificiel comprend également un dispositif d'actionnement pour ajuster la compression exercée par l'élément occlusif.

Il existe donc une liaison entre l'élément occlusif et le dispositif d'actionnement, qui dépend du mode d'actionnement dudit élément occlusif.

Par exemple, dans le cas d'un système occlusif hydraulique, l'élément occlusif est une manchette gonflable susceptible de contenir un volume ajustable d'un fluide et le dispositif d'actionnement comprend un réservoir de fluide, la liaison entre la manchette et le dispositif d'actionnement comprenant une tubulure permettant de transférer du fluide de manière bidirectionnelle de la manchette au réservoir selon que l'on souhaite augmenter ou diminuer la compression exercée.

Dans le cas d'un système occlusif électromécanique, l'élément occlusif se présente sous la forme d'une bande dont la tension est ajustable et le dispositif d'actionnement comprend un actionneur adapté pour ajuster une tension de la bande occlusive, la liaison entre la bande et le dispositif d'actionnement étant alors assurée par un câble ou tout autre moyen permettant d'appliquer une tension variable à la bande occlusive.

Dans les systèmes occlusifs les plus anciens, ce dispositif d'actionnement est commandé manuellement par le patient, par exemple par une pression exercée sur un dispositif de pompe agencé sous la peau.

A l'heure actuelle, des systèmes plus perfectionnés sont développés afin d'éviter au patient d'exercer une pression manuelle sur la pompe pour contrôler l'élément occlusif.

Le système occlusif comprend alors une unité de commande, également implantable dans le corps du patient, qui est adaptée pour commander le dispositif d'actionnement de la manchette.

Il existe actuellement différents types de systèmes occlusifs, mettant en oeuvre différentes technologies d'éléments occlusifs (hydraulique, mécanique, etc.) et différentes technologies d'actionnement associées (actionneur piézo-électrique, câbles, dispositifs à mémoire de forme, etc.).

Ces différents systèmes occlusifs sont connus de l'homme du métier.

S'agissant des sphincters urinaires artificiels, on pourra par exemple se référer aux documents suivants [1] à [4] cités plus haut ou encore aux documents [8] et [9].

L'invention n'est pas limitée à une technologie de système occlusif particulière.

Le dispositif d'actionnement et l'unité de commande sont avantageusement inclus dans un boîtier implantable dans le corps du patient destiné à les protéger.

Le boîtier est typiquement réalisé en un matériau biocompatible.

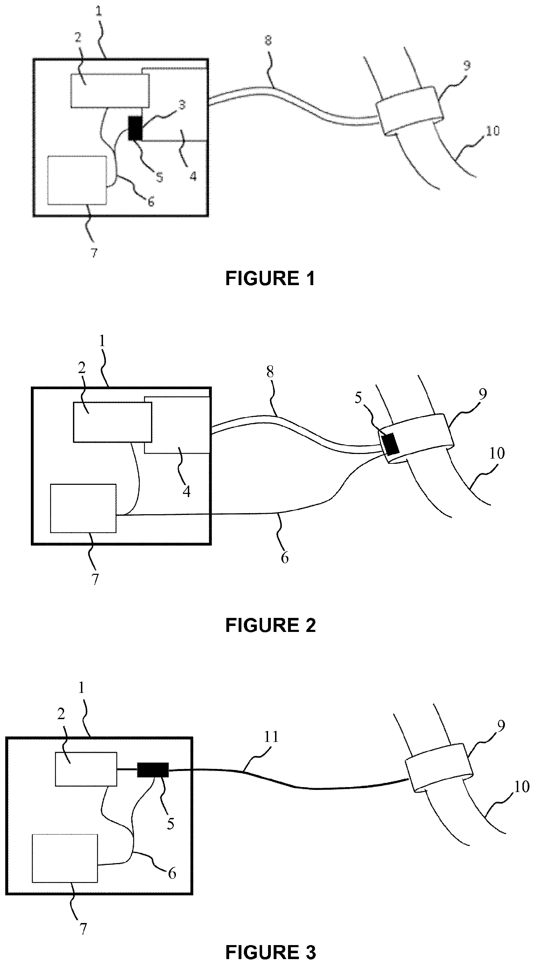

La figure 1 est un schéma de principe d'un système occlusif hydraulique 1 associé à un dispositif de détection de l'atrophie selon un mode de réalisation de l'invention.

L'élément occlusif 9 se présente sous la forme d'une manchette gonflable susceptible d'être remplie d'une quantité variable d'un fluide, une variation de la pression de fluide à l'intérieur de la manchette faisant varier la compression exercée sur le conduit naturel 10 à occlure.

Un réservoir 4 d'un fluide, par exemple du sérum physiologique, est agencé en liaison fluidique avec la manchette, par l'intermédiaire d'une tubulure 8.

L'ensemble de la manchette 9, du réservoir 4 et de la tubulure 2 forme le circuit hydraulique du système occlusif 1.

Ce circuit hydraulique permet de transférer une partie du fluide du réservoir dans la manchette, afin d'augmenter la compression exercée sur le conduit 10, et inversement de transférer une partie du fluide de la manchette vers le réservoir, pour diminuer la compression exercée par la manchette sur le conduit 10.

A cet effet, le système occlusif comprend en outre un dispositif d'actionnement 2 pour effectuer ce transfert de fluide et ainsi faire varier la compression exercée par la manchette sur le conduit 10.

De manière particulièrement avantageuse, le réservoir 4 présente un volume variable.

Par exemple, mais de manière non limitative, la variation de volume peut être réalisée en déplaçant une paroi du réservoir, le dispositif d'actionnement 2 comprenant un actionneur pour déplacer ladite paroi.

Ainsi, le réservoir peut comprendre une membrane roulante, un piston, un soufflet ou tout autre moyen permettant de faire varier son volume.

L'homme du métier est en mesure de sélectionner parmi les actionneurs existants un actionneur adéquat en fonction de l'implémentation envisagée vis-à-vis du réservoir.

On peut citer par exemple mais de manière non limitative un actionneur piézo-électrique, etc.

Bien qu'il ne soit pas illustré ici, le dispositif d'actionnement comprend un capteur permettant de mesurer l'action exercée sur le réservoir.

Par exemple, si l'actionnement consiste en un déplacement d'une paroi mobile du réservoir, ledit capteur peut consister en un capteur de position, permettant de déterminer la position de la paroi mobile.

Un calibrage permet de déterminer d'une part, la relation entre la position de la paroi mobile et la variation de volume du réservoir ; d'autre part, la relation entre la variation de volume du réservoir et la pression dans le circuit hydraulique et enfin entre la pression dans le circuit hydraulique et la compression exercée sur le conduit à occlure.

La relation entre la pression dans le circuit hydraulique et le volume transféré du réservoir vers la manchette gonflable peut éventuellement être exprimée sous la forme d'une relation mathématique.

Selon les cas, cette relation peut être linéaire ou non.

Ainsi, il est possible de déterminer le déplacement à imposer à la paroi mobile pour obtenir une pression donnée de fluide dans le circuit hydraulique en vue d'obtenir une compression donnée du conduit 10.

Dans ce cas, le contrôle en déplacement du dispositif d'actionnement est basé sur une mesure de la pression dans le circuit hydraulique.

A cet effet, dans le mode de réalisation illustré sur la figure 1, un capteur de pression 5 est agencé sur une paroi du réservoir 4 de manière à fournir une mesure de la pression de fluide dans le réservoir.

Le système occlusif 1 comprend en outre une unité de commande 7 adaptée pour solliciter le dispositif d'actionnement 2 de sorte à exercer une compression déterminée sur le conduit 10.

La liaison 6 entre l'unité de commande 7 et le dispositif d'actionnement 2 a été représentée sous forme filaire sur la figure 1, mais il va de soi qu'elle pourrait être mise en oeuvre sans fil, selon la technologie sélectionnée par l'homme du métier.

Il existe également une liaison 6 (filaire ou non) entre le capteur 5 et l'unité de commande 7.

La figure 2 illustre un autre mode de réalisation d'un système occlusif hydraulique 1.

Les composants désignés par les mêmes signes de référence que sur la figure 1 remplissent la même fonction et ne seront donc pas décrits en détail à nouveau.

Par rapport au dispositif illustré sur la figure 1, le capteur 5 permettant de mesurer la pression dans le circuit hydraulique n'est pas agencé sur une paroi du réservoir 4 mais sur la manchette occlusive 9, afin de mesurer directement la pression sur le conduit 10.

Il va de soi que l'on pourrait mettre en place un capteur de pression en tout autre endroit du système de compression sans pour autant sortir du cadre de la présente invention.

La figure 3 est un schéma de principe d'un système occlusif électromécanique 1 associé à un dispositif de détection de l'atrophie selon un mode de réalisation de l'invention.

L'élément occlusif 9 se présente sous la forme d'une bande disposée autour du conduit 10 à occlure, une variation de la tension de la bande faisant varier la compression exercée sur le conduit naturel 10 à occlure.

La bande est reliée par un élément 11 de transmission mécanique à un dispositif d'actionnement 2.

Par exemple, l'élément 11 est un câble.

Le dispositif d'actionnement est adapté pour faire varier la course et/ou la tension de l'élément 11 de transmission mécanique, en vue d'augmenter ou de diminuer la compression exercée sur le conduit 10 par la bande occlusive 9.

L'homme du métier est en mesure de sélectionner parmi les actionneurs existants un actionneur adéquat en fonction de l'élément de transmission mécanique choisi.

On peut citer par exemple mais de manière non limitative un actionneur piézo-électrique, etc.

Un calibrage permet de déterminer d'une part, la relation entre la course et la tension de l'élément de transmission mécanique et, d'autre part, la relation entre la tension de la bande occlusive et la compression exercée sur le conduit à occlure.

La relation entre la course et la tension de l'élément de transmission mécanique peut éventuellement être exprimée sous la forme d'une relation mathématique.

Selon les cas, cette relation peut être linéaire ou non.

Ainsi, il est possible de déterminer la course à imposer à l'élément de transmission mécanique pour obtenir une tension donnée de la bande occlusive en vue d'obtenir une compression donnée du conduit 10.

Dans ce cas, le contrôle en déplacement du dispositif d'actionnement est basé sur une mesure de la tension de l'élément de transmission mécanique.

A cet effet, comme illustré sur la figure 3, un capteur de tension 5 est agencé sur l'élément de transmission mécanique de manière à fournir une mesure de la tension dudit élément.

Le système occlusif comprend en outre une unité de commande 7 adaptée pour solliciter le dispositif d'actionnement 2 de sorte à exercer une compression déterminée sur le conduit 10.

La liaison 6 entre l'unité de commande 7 et le dispositif d'actionnement 2 a été représentée sous forme filaire sur la figure 3, mais il va de soi qu'elle pourrait être mise en oeuvre sans fil, selon la technologie sélectionnée par l'homme du métier.

Il existe également une liaison 6 (filaire ou non) entre le capteur 5 et l'unité de commande 7.

Dans un autre mode de réalisation, le système occlusif est basé sur une pompe péristaltique permettant de transférer un fluide contenu dans un réservoir de ou vers la manchette occlusive.

Les modes de réalisation décrits ci-dessus ne sont pas destinés à limiter l'invention et l'on pourra choisir d'autres moyens d'actionnement et d'autres capteurs sans sortir de la portée de la présente invention.

### Mesure de la compression exercée sur le conduit à occlure

La mesure de la compression exercée sur le conduit à occlure peut être réalisée directement sur ledit conduit ou, de manière préférée car généralement plus aisée à mettre en oeuvre, indirectement, par une grandeur mesurée au niveau de l'élément occlusif, du dispositif d'actionnement ou de la liaison entre l'élément occlusif et le dispositif d'actionnement.

Ainsi, comme déjà indiqué plus haut, la pression dans le circuit hydraulique (mesurée au niveau d'une paroi du réservoir ou au niveau de la manchette) ou la tension de la bande occlusive dans le cas d'un système électromécanique, permet de fournir une indication de la compression du conduit.

La relation entre le niveau de compression du conduit et la pression dans le circuit hydraulique ou la tension de la bande occlusive est établie au préalable, en fonction du mode d'actionnement et des matériaux retenus lors de la conception du dispositif.

Un calibrage de cette relation peut en plus être effectué dans certains cas de figure en effectuant des mesures de pression dans le conduit à occlure à différentes pressions dans le circuit hydraulique, ou à différentes tensions, selon le mode de réalisation sélectionné.

### Paramètre représentatif

Par ailleurs, on détermine un paramètre représentatif d'une sollicitation à appliquer au dispositif d'actionnement pour obtenir une compression déterminée du conduit à occlure.

Ainsi, dans le cas d'un système occlusif hydraulique tel que décrit ci-dessus, le paramètre représentatif peut être défini comme étant la position de la paroi mobile du réservoir par rapport à une position de référence pour obtenir une compression déterminée du conduit.

Par exemple, la position de référence peut être la position de la paroi dans laquelle aucune compression n'est exercée sur le conduit.

Cette position de référence peut ainsi être considérée comme une position en butée initiale correspondant à une manchette occlusive vide.

Le paramètre représentatif peut être défini comme la position de la paroi mobile dans laquelle une compression donnée du conduit est atteinte.

Le paramètre représentatif peut également être défini comme étant la distance à parcourir par la paroi mobile du réservoir pour passer de la position en butée initiale à la position permettant d'atteindre ladite compression donnée du conduit à occlure.

De manière alternative, le paramètre représentatif peut être défini comme étant le volume de fluide dans le réservoir pour obtenir une compression maximale du conduit, le volume de référence étant le volume maximal du réservoir.

Dans le cas d'un système occlusif électromécanique, le paramètre représentatif peut être défini comme la course à imposer à l'élément de transmission mécanique pour obtenir une tension correspondant à une compression donnée du conduit à occlure.

Dans le cas d'un système occlusif basé sur une pompe péristaltique, le paramètre représentatif peut être défini comme la course angulaire effectuée par la pompe par rapport à une position de référence pour obtenir une compression donnée du conduit.

On peut noter que, quel que soit le type de système occlusif, la valeur du paramètre représentatif peut varier selon la condition du patient, par exemple selon sa posture.

Le paramètre représentatif pourra donc être défini pour une condition déterminée du patient et être surveillé au cours du temps en n'étant enregistré que lorsque le patient est dans la condition adéquate.

### Surveillance du paramètre représentatif au cours du temps

En cas d'atrophie du conduit à occlure, une dérive du paramètre représentatif du patient se produit.

Cette dérive est due au fait que, compte tenu de l'atrophie, l'épaisseur de la paroi du conduit diminue.

Par conséquent, pour exercer une même compression donnée du conduit, il est nécessaire d'appliquer une sollicitation plus importante au dispositif d'actionnement.

Par exemple, il est nécessaire d'apporter davantage de fluide dans la manchette (dans le cas d'un système hydraulique) ou d'appliquer un déplacement plus grand au câble lié à la bande occlusive (dans le cas d'un système électromécanique).

La surveillance du paramètre représentatif au cours du temps permet donc de détecter, par le biais d'une dérive de la valeur de ce paramètre, une atrophie du conduit à occlure.

Le paramètre représentatif est donc surveillé au cours du temps et enregistré dans une mémoire de l'unité de traitement.

De manière particulièrement avantageuse, ledit paramètre est mesuré lorsque le patient est dans une condition prédéterminée, ou lors de la transition d'une première condition prédéterminée à une seconde condition prédéterminée du patient.

Par « condition prédéterminée », on entend une posture particulière (par exemple la station debout, couchée ou assise) et/ou la réalisation d'une action particulière (par exemple la miction, dans le cas d'un sphincter urinaire artificiel).

Par exemple, lorsque le patient est allongé et immobile, le système peut réduire la compression exercée sur le conduit à occlure.

Tel est le cas par exemple dans un sphincter urinaire artificiel mettant en oeuvre un modèle prédictif des fuites urinaires tel que décrit dans [1], dans lequel la compression exercée sur le conduit est ajustée en fonction de l'activité du patient : l'application d'une faible compression de l'urètre est en effet suffisante lorsque le patient est endormi.

Dans ce cas particulier, le paramètre représentatif d'une sollicitation à appliquer peut être le volume à injecter dans la manchette (dans le cas d'un système hydraulique) ou la course à appliquer au câble (dans le cas d'un système électromécanique) pour passer d'une compression donnée correspondant à la situation « patient allongé » à une compression donnée plus importante correspondant à la situation « patient debout», les valeurs des dites compressions étant configurées par l'utilisateur.

Dans un autre cas, la mesure du paramètre représentatif peut être effectuée lors de la miction.

Le patient peut alors être équipé d'un ou plusieurs capteurs permettant de détecter la ou les conditions prédéterminées mentionnées ci-dessus.

A titre d'exemple non limitatif, pour déterminer la posture du patient, on pourra utiliser un accéléromètre, un gyroscope et/ou un inclinomètre.

L'homme du métier est à même de définir le(s) capteur(s) adapté(s) pour détecter une ou des conditions prédéterminées du patient.

### Critère de détection d'une atrophie

Le critère de détection d'une atrophie peut être sélectionné parmi différentes possibilités, dont certaines seront décrites ci-dessous.

Selon une première forme d'exécution de l'invention, le critère de détection d'une atrophie est rempli lorsque le paramètre représentatif est supérieur à une valeur fixe, indépendante du patient, fixée arbitrairement.

Selon une autre forme d'exécution, le critère de détection d'une atrophie est rempli lorsque le paramètre représentatif est supérieur à un pourcentage d'une valeur du paramètre mesurée initialement.

Par exemple, on mesure la valeur du paramètre représentatif après l'implantation du système occlusif chez le patient, et l'on choisit cette valeur comme référence.

On choisit également un pourcentage de cette valeur de référence comme étant la valeur au-delà de laquelle on détecte une atrophie.

Par exemple, ce pourcentage pourrait être de l'ordre de 130 à 180%.

Un avantage de ce critère est qu'il permet de tenir compte de la situation individuelle du patient, puisqu'il est basé sur une mesure faite sur le patient à l'initialisation du procédé de détection.

Selon une autre forme d'exécution de l'invention, le critère de détection d'une atrophie implique la comparaison, non du paramètre représentatif lui-même, mais d'une fonction dudit paramètre, avec une valeur déterminée.

Ainsi, la valeur à comparer avec ladite valeur déterminée peut être une valeur issue d'une fonction mathématique construite à partir d'une base de données de valeurs dudit paramètre représentatif mesurées périodiquement au cours du temps.

Une atrophie pouvant évoluer lentement dans un premier temps puis plus rapidement, un critère sur l'évolution de l'atrophie des tissus dans le temps peut être avantageux.

A titre d'exemple non limitatif, en fixant un critère basé sur la dérivée de la courbe d'évolution du paramètre représentatif, il est possible de prévenir le clinicien d'une atrophie lorsque celle-ci évolue rapidement, c'est-à-dire lorsque cette dérivée atteint ou dépasse une valeur déterminée.

Il est par ailleurs envisageable de définir un critère de décision plus complexe, qui prenne en compte simultanément différentes conditions en combinant différents critères dont ceux notamment présentés dans les paragraphes précédents.

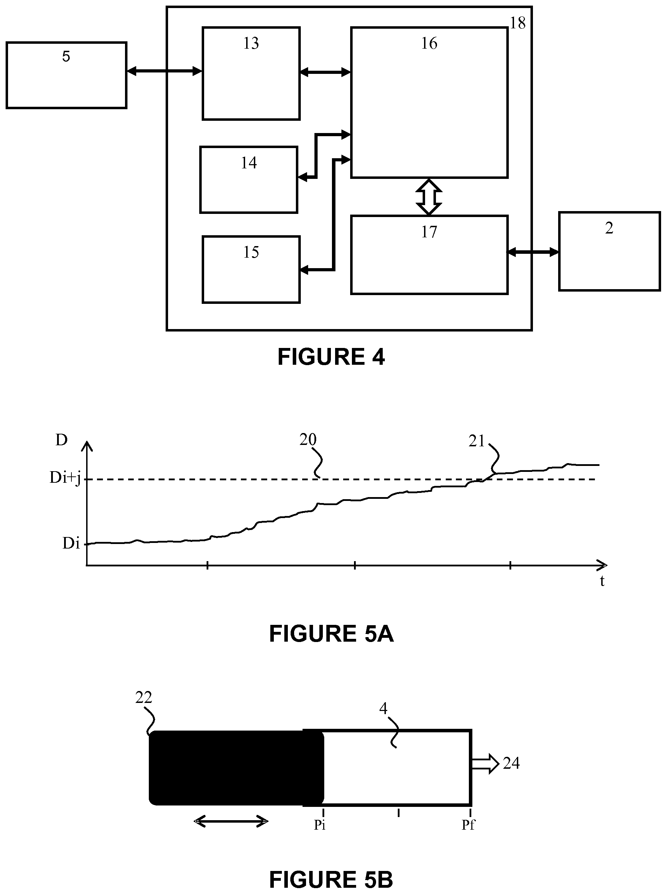

La figure 4 illustre une forme d'exécution de l'architecture générale de l'unité de traitement permettant la détection d'une éventuelle atrophie.

L'unité de traitement 18 comprend un microprocesseur 16 adapté pour implémenter un algorithme permettant de déterminer, à partir des mesures du (des) capteur(s), la valeur du paramètre représentatif.

A cet effet, le microprocesseur 16 communique avec au moins un capteur 5 au moyen d'une interface 13.

La communication est schématisée par des flèches et peut être réalisée par une liaison filaire ou par une liaison sans fil, selon des protocoles connus.

L'unité de traitement 18 comprend en outre une mémoire 14 dans laquelle sont enregistrés le programme de détection, les valeurs du paramètre représentatif, ainsi que les conditions à remplir pour qu'une atrophie soit détectée.

L'unité de traitement comprend en outre une ou plusieurs horloges 15.

Le microprocesseur 16 est relié à l'interface 13, la mémoire 14 et l'horloge 15.

Le microprocesseur 16 communique en outre avec le dispositif d'actionnement 2 par l'intermédiaire d'une interface 17.

Les figures 5A et 5B illustrent un exemple de détection d'une atrophie dans le cas d'un système occlusif hydraulique.

Dans le mode de réalisation représenté sur la figure 5B, le réservoir 4 a un volume variable grâce au déplacement d'un piston 22.

La course du piston est définie par deux positions limites : la position Pi, correspondant à une butée initiale du piston dans laquelle la manchette occlusive est vide, et la position Pf, correspondant à une butée finale du piston dans laquelle la manchette occlusive est remplie de fluide de sorte à exercer une compression maximale du piston.

La flèche 24 indique la direction de sortie du fluide du réservoir vers la manchette occlusive.

La position du piston permettant d'exercer une compression donnée sur le conduit est comprise entre les positions Pi et Pf.

Dans ce cas, c'est la distance entre ladite position et une position de référence correspondant à la position Pi qui est définie comme le paramètre représentatif de la sollicitation à appliquer au dispositif d'actionnement pour obtenir une compression donnée du conduit.

On désigne par Di la distance initiale.

Le graphe de la figure 5A présente l'évolution 21 de la distance D au cours du temps, dans le cas où une atrophie se produit.

Comme on peut le voir sur ce graphe, la distance D croît progressivement à partir de la valeur initiale Di.

Lorsque la courbe de la distance D atteint un seuil prédéterminé 20 correspondant à une valeur de distance Di+j, on considère qu'une atrophie est détectée.

Le cas échéant, une alerte est émise par l'unité de traitement à l'attention du patient et/ou du praticien.

### Détection d'une éventuelle fuite lente dans le circuit hydraulique

Dans le cas d'un système occlusif hydraulique, une fuite lente se produisant dans le circuit hydraulique est susceptible de modifier au cours du temps la sollicitation à appliquer au dispositif d'actionnement pour obtenir une compression déterminée du conduit à occlure.

Par « fuite lente », on entend dans le présent texte une perte de liquide en petite quantité et s'étalant sur une longue plage de temps (au moins plusieurs jours, mais plus généralement de l'ordre de plusieurs mois), occasionnant une diminution progressive de la pression dans le circuit hydraulique.

Par opposition, une fuite est considérée comme rapide si elle cause une diminution brutale de la pression dans le circuit hydraulique.

Ainsi, une fuite rapide est détectable dès que l'événement déclencheur a eu lieu (par exemple, une déconnexion de la tubulure reliant la manchette au réservoir de fluide) ou peu après (par exemple, endommagement important de l'un des matériaux assurant l'étanchéité).

Au contraire, une fuite lente ne devient détectable que plusieurs jours, voire plusieurs mois après la survenance de la défaillance qui en est la cause.

De telles fuites lentes peuvent avoir différentes causes, parmi lesquelles :
- l'endommagement d'un élément mécanique du circuit hydraulique, par exemple une fissuration d'un élément,
- un défaut de la connexion de la tubulure au réservoir ou à la manchette occlusive,
- la porosité du matériau constituant la tubulure et/ou la manchette occlusive (qui sont généralement en silicone) et une concentration inadéquate de la solution saline contenue dans le circuit hydraulique, générant un gradient de concentration entre le milieu extracellulaire et le circuit hydraulique, qui entraîne une diffusion d'eau du milieu le moins concentré vers le milieu le plus concentré. En particulier, si le fluide contenu dans le circuit hydraulique est trop peu concentré (hypotonique) par rapport au milieu extérieur, l'eau tendra à diffuser vers l'extérieur du circuit hydraulique, conduisant à une diminution du volume dans ledit circuit.

Ainsi, si le paramètre représentatif est une distance à parcourir par la paroi mobile du réservoir pour exercer une compression donnée sur le conduit, une fuite lente aura pour effet que cette distance augmente au cours du temps.

Cette dérive a pour effet de détecter une variation du paramètre représentatif défini plus haut, sans qu'une atrophie ne se soit effectivement produite, ce qui entraîne une fausse détection.

Pour remédier à cet éventuel problème (qui ne concerne que les systèmes occlusifs hydrauliques), l'invention propose, dans une forme particulière de réalisation, de détecter une éventuelle fuite lente, ce qui permet de distinguer une fuite lente d'une éventuelle atrophie.

A cet effet, on implémente le procédé décrit ci-dessous.

D'une manière générale, la détection d'une fuite lente implique la surveillance de la pression dans le circuit hydraulique au cours du temps et la comparaison de ladite pression ou d'une fonction de ladite pression avec un critère prédéterminé.

### Mesure de la pression dans le circuit hydraulique

La mesure de la pression dans le circuit hydraulique peut être réalisée en tout point dudit circuit, par exemple avec un ou plusieurs des capteurs de pression mentionnés plus haut.

Dans la présente invention, on s'intéresse à l'évolution de la pression dans le circuit hydraulique dans une situation particulière, correspondant à une sollicitation déterminée du dispositif d'actionnement.

Cette sollicitation déterminée dépend du type de dispositif d'actionnement mis en oeuvre dans le système occlusif.

Par exemple, lorsque le réservoir comprend une paroi mobile permettant de faire varier son volume et que le dispositif d'actionnement est adapté pour déplacer ladite paroi d'une distance déterminée, la sollicitation déterminée dans laquelle on surveille la pression dans le circuit hydraulique peut correspondre à une position de butée ouverte de ladite paroi (correspondant à un volume maximal du réservoir, la manchette occlusive étant alors vide).

Lorsqu'il se produit une fuite lente dans le circuit hydraulique, la pression dans le circuit hydraulique pour cette position déterminée de la paroi tend à diminuer, et peut même devenir négative.

De manière similaire, dans le cas où le dispositif d'actionnement comprend une pompe péristaltique, la sollicitation pour laquelle on surveille l'évolution de la pression dans le circuit hydraulique peut être définie pour une position de référence de ladite pompe permettant d'obtenir une compression donnée du conduit.

### Surveillance de la pression au cours du temps

Pour détecter une éventuelle fuite lente dans le circuit hydraulique, on enregistre périodiquement la pression dans le circuit hydraulique pour la sollicitation prédéterminée décrite ci-dessus.

La périodicité de mesure n'est pas nécessairement régulière, c'est-à-dire qu'il peut s'écouler des intervalles de temps de différentes longueurs entre deux mesures consécutives.

Par ailleurs, l'enregistrement de la pression n'est pas nécessairement effectué à chaque fois que la sollicitation prédéterminée est rencontrée, mais peut éventuellement être réalisé moins souvent, selon une fréquence prédéterminée

D'autre part, la périodicité de mesure peut dépendre du type de système occlusif considéré.

Par exemple, pour un système occlusif urinaire ou anal, on procède au moins une fois par jour à une ouverture de la manchette occlusive pour permettre la miction ou la défécation.

Par conséquent, pour ces systèmes, on peut surveiller la pression dans le circuit hydraulique quotidiennement, en enregistrant la pression dans le circuit hydraulique lors d'au moins une miction ou défécation.

Dans d'autres systèmes (par exemple les anneaux gastriques), le système peut se calibrer soit lors d'une commande effectuée par l'utilisateur, soit de manière autonome en plaçant le dispositif d'actionnement dans une position de référence, dans des conditions qui n'affectent pas la fonction du système occlusif.

### Critère de détection d'une fuite lente

Le critère de détection d'une fuite lente peut être sélectionné parmi différentes possibilités, dont certaines seront décrites ci-dessous.

Selon une première forme d'exécution de l'invention, le critère de détection d'une telle fuite lente est rempli lorsque la pression dans le circuit hydraulique devient inférieure à une valeur fixe, indépendante du patient, fixée arbitrairement.

Cette valeur seuil est de préférence nulle ou négative.

Selon une autre forme d'exécution, le critère de détection d'une fuite lente est rempli lorsque la pression dans le circuit hydraulique devient inférieure à un pourcentage de la valeur de la pression mesurée initialement.

Par exemple, on mesure la valeur de la pression dans le circuit hydraulique pour une sollicitation déterminée du dispositif d'actionnement après l'implantation du système occlusif chez le patient, et l'on choisit cette valeur comme référence.

On choisit également un pourcentage de cette valeur de référence comme étant la valeur en-deçà de laquelle on détecte une fuite lente.

Par exemple, ce pourcentage pourrait être de l'ordre de 20 %.

Cette valeur peut être également négative et proportionnelle à un ou plusieurs paramètres du système.

Un avantage de ce critère est qu'il permet de tenir compte de la situation individuelle du patient, puisqu'il est basé sur une mesure faite sur le patient à l'initialisation du procédé de détection.

Selon une autre forme d'exécution de l'invention, le critère de détection d'une fuite lente implique la comparaison, non de la pression mesurée elle-même, mais d'une fonction de ladite pression, avec une valeur déterminée.

Ainsi, cette valeur à comparer à ladite valeur déterminée peut être une valeur issue d'une fonction mathématique construite à partir d'une base de données de valeurs de la pression mesurées périodiquement au cours du temps.

Ainsi, on peut par exemple considérer la présence d'une fuite lente lorsque le produit x.P passe sous une valeur seuil, x étant un paramètre pouvant évoluer dans le temps afin de prendre en compte le vieillissement des matériaux du système occlusif, et P la pression mesurée dans le circuit hydraulique pour une sollicitation donnée.

Par exemple, cette valeur seuil peut en particulier être négative.

Il est par ailleurs envisageable de définir un critère de détection plus complexe, qui prenne en compte simultanément différentes conditions en combinant différents critères dont ceux notamment présentés dans les paragraphes précédents.

D'une manière générale, une valeur seuil négative présente l'avantage de distinguer avec certitude une atrophie d'une fuite lente.

En effet, en présence d'une atrophie, même s'il se produit une variation de la sollicitation à appliquer au dispositif d'actionnement pour obtenir une compression déterminée du conduit, le volume global de fluide dans le circuit hydraulique reste sensiblement le même et la pression dans le circuit hydraulique restera toujours positive pour une sollicitation déterminée du dispositif d'actionnement.

L'observation d'une pression inférieure à une valeur seuil nulle ou négative dans le circuit hydraulique pour une sollicitation déterminée du dispositif d'actionnement est donc caractéristique d'une fuite lente.

Ce procédé peut être mis en oeuvre dans l'unité de traitement illustrée sur la figure 4.

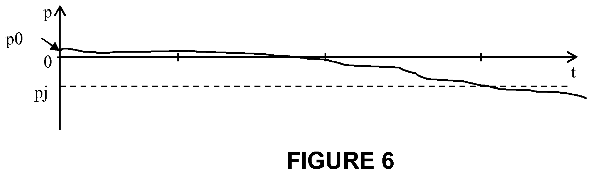

La figure 6 illustre un exemple de détection d'une fuite lente dans un système occlusif hydraulique.

Comme dans le mode de réalisation représenté sur la figure 5B, le réservoir 4 a un volume variable grâce au déplacement d'un piston 22.

La course du piston est définie par deux positions limites : la position Pi, correspondant à une butée initiale du piston dans laquelle la manchette occlusive est vide, et la position Pf, correspondant à une butée finale du piston dans laquelle la manchette occlusive est remplie de fluide de sorte à exercer une compression maximale du conduit.

On s'intéresse à la variation de la pression dans le circuit hydraulique lorsque le piston est dans la position Pi.

La pression dans le circuit hydraulique à l'initialisation du système occlusif est notée p0.

Le graphe de la figure 6 présente l'évolution de la pression p au cours du temps lorsque le piston est dans la position Pi, dans le cas où une fuite lente se produit.

Comme on peut le voir sur ce graphe, la pression p décroît progressivement à partir de la valeur initiale p0.

Lorsque la courbe de la pression p atteint un seuil prédéterminé noté pj, on considère qu'une fuite lente est détectée.

Il est ainsi possible de différencier une fuite lente du système d'une atrophie du conduit naturel.

Le cas échéant, une alerte est émise par l'unité de traitement à l'attention du patient et/ou du praticien.

Le praticien peut alors décider d'ajouter un nouveau volume de fluide dans le circuit hydraulique, ce qui permet d'éviter une nouvelle intervention chirurgicale.

Cet ajout peut être fait au travers d'un port d'injection qui est généralement prévu sur le circuit hydraulique, en particulier sur le réservoir.

Le port d'injection peut comprendre un septum agencé dans l'une des parois du réservoir qui est placée en regard de la peau du patient, de manière à ce que le praticien puisse introduire, à travers la peau, une aiguille dans le réservoir.

Le septum est en un matériau biocompatible permettant d'assurer l'étanchéité du réservoir pendant et après le retrait de l'aiguille.

Le silicone est généralement employé pour cet usage.

### REFERENCES

[1] WO 2009/027196
[2] Development of a Novel Artificial Urinary Sphincter, H. Lamraoui et al, IEEE/ASME Transactions on Mechatronics, Vol. 15, No. 6, Dec. 2010
[3] US 6,162,238
[4] US 5,704,893
[5] F. Maillet, J.-M. Buzelin, O. Bouchot, and G. Karam, "Management of artificial 1 urinary sphincter dysfunction," European Urology, vol. 46, no. 2, pp. 241-246, Aug. 2004.
[6] C. Hajivassiliou, "A review of the complications and results of implantation of the AMS artificial urinary sphincter," European Urology, vol. 35, no. 1, pp. 36-44, 1999.
[7] D. K. Montague and K. W. Angermeier, "Artificial urinary sphincter troubleshooting," Urology, vol. 58, no. 5, pp. 779-782, Nov. 2001.
[8] US 5,509,888
[9] US 6,135,945

## Revendications

1. Système occlusif implantable dans le corps humain ou animal pour occlure un conduit naturel (10) du corps humain ou animal, comprenant :
- un élément occlusif (9) destiné à entourer une partie du conduit naturel (10) à occlure,
- un dispositif d'actionnement (2) dudit élément occlusif (10) pour faire varier la compression exercée par ledit élément occlusif (9) sur ledit conduit (10),
- une unité de commande (7) adaptée pour solliciter le dispositif d'actionnement (2) de sorte à exercer une compression déterminée sur le conduit (10), et
- un dispositif de détection de l'atrophie dudit conduit naturel (10), comprenant :
(i) un capteur adapté pour mesurer la compression exercée par l'élément occlusif (9) sur le conduit naturel (10), et
(ii) une unité de traitement adaptée pour :
• déterminer un paramètre représentatif d'une sollicitation à appliquer au dispositif d'actionnement (2) pour obtenir une compression déterminée du conduit (10) par l'élément occlusif (9),
• surveiller ledit paramètre au cours du temps, et
• détecter une atrophie du conduit naturel (10) lorsque ledit paramètre représentatif remplit un critère prédéterminé.

2. Système selon la revendication 1, dans lequel ledit critère prédéterminé est choisi parmi l'une des conditions suivantes ou une combinaison desdites conditions :
- le paramètre représentatif est supérieur à une valeur fixe,
- le paramètre représentatif est supérieur à un pourcentage d'une valeur dudit paramètre représentatif mesurée initialement,
- une valeur issue d'une fonction mathématique construite à partir d'une base de données de valeurs dudit paramètre représentatif mesurées périodiquement au cours du temps est supérieure à une valeur déterminée.

3. Système selon l'une des revendications 1 ou 2, ledit système étant un système occlusif hydraulique dans lequel :
- l'élément occlusif (9) est une manchette gonflable comprenant un volume variable d'un fluide,
- le dispositif d'actionnement (2) comprend un réservoir (4) contenant un fluide, en liaison fluidique avec ladite manchette gonflable,
la variation de la compression exercée par la manchette sur le conduit (10) étant réalisée par transfert d'un volume ajustable dudit fluide entre le réservoir (4) et la manchette.

4. Système selon la revendication 3, dans lequel le capteur est adapté pour mesurer la compression du conduit naturel directement entre l'élément occlusif (9) et le conduit naturel (10).

5. Système selon la revendication 3, dans lequel le capteur est adapté pour mesurer la pression dans une région dudit circuit hydraulique et l'unité de traitement est configurée pour déduire de ladite mesure la compression exercée par la manchette sur le conduit naturel (10).

6. Système selon l'une des revendications 3 à 5, dans lequel ledit paramètre représentatif est le volume de fluide à transférer du réservoir (4) dans la manchette gonflable pour atteindre une compression du conduit (10) déterminée.

7. Système selon les revendications 3 à 6, dans lequel l'unité de traitement comprend une mémoire dans laquelle est enregistrée une relation entre la pression dans le circuit hydraulique et le volume transféré dans la manchette gonflable.

8. Système selon l'une des revendications 3 à 7, dans lequel le réservoir (4) est un réservoir à volume variable comprenant une paroi mobile et en ce que l'unité de traitement est configurée pour déterminer le volume de fluide transféré à partir d'une mesure du déplacement de ladite paroi mobile.

9. Système selon l'une des revendications 3 à 8, comprenant en outre des moyens de détection d'une fuite lente dans le circuit hydraulique, lesdits moyens étant configurés pour :
- la mesure de l'évolution de la pression dans le circuit hydraulique pour une sollicitation déterminée du dispositif d'actionnement (2),
- la détection d'une fuite lente dans le circuit hydraulique lorsque la pression mesurée dans ledit circuit pour ladite sollicitation déterminée du dispositif d'actionnement (2) remplit un critère prédéterminé.

10. Système selon la revendication 9, **caractérisé en ce que** ledit critère prédéterminé de détection d'une fuite lente est choisi parmi l'une des conditions suivantes ou une combinaison desdites conditions :
- la pression dans le circuit hydraulique est inférieure à une valeur fixe,
- la pression dans le circuit hydraulique est inférieure à un pourcentage d'une valeur de la pression mesurée initialement pour ladite sollicitation déterminée du dispositif d'actionnement, et
- une valeur issue d'une fonction mathématique construite à partir d'une base de données de valeurs de ladite pression pour ladite sollicitation du dispositif d'actionnement, ladite pression étant enregistrée périodiquement au cours du temps, est inférieure à une valeur déterminée.

11. Système selon l'une des revendications 1 ou 2, ledit système étant un système occlusif mécanique dans lequel :
- l'élément occlusif (9) est une bande de longueur variable entourant le conduit naturel (10),
- le dispositif d'actionnement (2) comprend un élément de transmission mécanique (11) reliant ladite bande et un actionneur adapté pour ajuster la course dudit élément de transmission mécanique,
la variation de la compression exercée par la bande sur le conduit (10) étant réalisée par l'ajustement de la course dudit élément de transmission mécanique.

12. Système selon la revendication 11, dans lequel le capteur est adapté pour mesurer la tension mécanique dudit élément de transmission mécanique et en ce que l'unité de traitement est configurée pour déduire de ladite tension mécanique mesurée la compression exercée par la bande sur le conduit naturel (10).

13. Système selon l'une des revendications 11 ou 12, dans lequel ledit paramètre représentatif est la course dudit élément de transmission mécanique entre l'actionneur et la bande occlusive pour atteindre une compression du conduit (10) déterminée.

14. Système selon l'une des revendications 11 à 13, dans lequel l'unité de traitement comprend une mémoire dans laquelle est enregistrée une relation entre la course de l'élément de transmission mécanique et la tension mécanique dudit élément de transmission ou la compression exercée par la bande.

15. Système selon l'une des revendications 1 à 14, **caractérisé en ce que** ledit paramètre représentatif est défini pour une condition déterminée du patient et **en ce que** ledit système occlusif comprend un capteur adapté pour détecter ladite condition déterminée du patient.

16. Système selon l'une des revendications 1 à 15, dans lequel l'unité de traitement est incluse dans l'unité de commande (7) du système occlusif.

17. Système selon l'une des revendications 1 à 16, comprenant en outre des moyens d'émission d'une alarme à un utilisateur si le critère de détection d'une atrophie du conduit naturel est rempli.

18. Système occlusif selon l'une des revendications 1 à 17, consistant en un sphincter urinaire artificiel.

## Patentansprüche

1. Implantierbares Verschlusssystem in den Körper eines Menschen oder eines Tiers zum Verschließen einer natürlichen Leitung (10) des menschlichen oder tierischen Körpers, umfassend:
- ein Verschlusselement (9), das bestimmt ist, einen Teil der zu verschließenden natürlichen Leitung (10) zu umschließen,
- eine Betätigungsvorrichtung (2) des Verschlusselements (10), um die von dem Verschlusselement (9) auf die Leitung (10) ausgeübte Kompression zu variieren,
- eine Steuereinheit (7), die ausgebildet ist, um die Betätigungsvorrichtung (2) derart zu beanspruchen, dass eine bestimmte Kompression auf die Leitung (10) ausgeübt wird, und
- eine Erfassungsvorrichtung der Atrophie der natürlichen Leitung (10), umfassend:
(i) einen Sensor, der ausgebildet ist, um die von dem Verschlusselement (9) auf die natürliche Leitung (10) ausgeübte Kompression zu messen, und
(ii) eine Verarbeitungseinheit, die ausgebildet ist, um:
• einen Parameter zu bestimmen, der für eine auf die Betätigungsvorrichtung (2) auszuübende Beanspruchung repräsentativ ist, um eine bestimmte Kompression der Leitung (10) durch das Verschlusselement (9) zu erhalten,
• den Parameter im Laufe der Zeit zu überwachen, und
• eine Atrophie der natürlichen Leitung (10) zu ermitteln, wenn der repräsentative Parameter ein vorbestimmtes Kriterium erfüllt.

2. System nach Anspruch 1, wobei das vorbestimmte Kriterium aus einer der folgenden Bedingungen oder einer Kombination der Bedingungen ausgewählt ist:
- der repräsentative Parameter liegt über einem festen Wert,
- der repräsentative Parameter liegt über einem Prozentsatz eines Wertes des anfänglich gemessenen repräsentativen Parameters,
- ein Wert, ausgegeben von einer mathematischen Funktion, konstruiert auf der Basis einer Datenbank von periodisch im Laufe der Zeit gemessenen Werten des repräsentativen Parameters, liegt über einem vorbestimmten Wert.

3. System nach einem der Ansprüche 1 oder 2, wobei das System ein hydraulisches Verschlusssystem ist, wobei:
- das Verschlusselement (9) eine aufblasbare Manschette ist, die ein variables Volumen eines Fluids umfasst,
- die Betätigungsvorrichtung (2) ein Reservoir (4) umfasst, das ein Fluid enthält, in fluidischer Verbindung mit der aufblasbaren Manschette,
wobei die Variation der von der Manschette auf die Leitung (10) ausgeübten Kompression durch Transfer eines einstellbaren Volumens des Fluids zwischen dem Reservoir (4) und der Manschette durchgeführt wird.

4. System nach Anspruch 3, wobei der Sensor ausgebildet ist, um die Kompression der natürlichen Leitung direkt zwischen dem Verschlusselement (9) und der natürlichen Leitung (10) zu messen.

5. System nach Anspruch 3, wobei der Sensor ausgebildet ist, um den Druck in einer Region des Hydraulikkreises zu messen und die Verarbeitungseinheit konfiguriert ist, um aus der Messung die von der Manschette auf die natürliche Leitung (10) ausgeübte Kompression abzuleiten.

6. System nach einem der Ansprüche 3 bis 5, wobei der repräsentative Parameter das Volumen des Fluids ist, das vom Reservoir (4) in die aufblasbare Manschette zu transferieren ist, um eine bestimmte Kompression der Leitung (10) zu erhalten.

7. System nach einem der Ansprüche 3 bis 6, wobei die Verarbeitungseinheit einen Speicher umfasst, in welchem ein Verhältnis zwischen dem Druck im Hydraulikkreis und dem in die aufblasbare Manschette transferierten Volumen gespeichert ist.

8. System nach einem der Ansprüche 3 bis 7, wobei das Reservoir (4) ein Reservoir mit variablen Volumen ist, umfassend eine bewegbare Wand, und die Verarbeitungseinheit konfiguriert ist, um das transferierte Fluidvolumen ausgehend von einer Messung der Verlagerung der bewegbaren Wand zu bestimmen.

9. System nach einem der Ansprüche 3 bis 8, umfassend ferner Detektionsmittel eines langsamen Verlusts im Hydraulikkreis, wobei die Mittel konfiguriert sind für:
- die Messung der Entwicklung des Drucks im Hydraulikkreis für eine bestimmte Beanspruchung der Betätigungsvorrichtung (2),
- die Detektion eines langsamen Verlusts im Hydraulikkreis, wenn der in dem Kreis für die bestimmte Beanspruchung der Betätigungsvorrichtung (2) gemessene Druck ein vorbestimmtes Kriterium erfüllt.

10. System nach Anspruch 9, **dadurch gekennzeichnet, dass** das vorbestimmte Kriterium der Detektion eines langsamen Verlusts aus einer der folgenden Bedingungen oder einer Kombination der Bedingungen ausgewählt ist:
- der Druck im Hydraulikkreis liegt unter einem festen Wert,
- der Druck im Hydraulikkreis liegt unter einem Prozentsatz eines Wertes des anfänglich für die bestimmte Beanspruchung der Betätigungsvorrichtung gemessenen Drucks, und
- ein Wert, ausgegeben von einer mathematischen Funktion, konstruiert auf der Basis einer Datenbank von Druckwerten für die Beanspruchung der Betätigungsvorrichtung, wobei der Druck periodisch gespeichert wird, liegt unter einem vorbestimmten Wert.

11. System nach einem der Ansprüche 1 oder 2, wobei das System ein mechanisches Verschlusssystem ist, wobei:
- das Verschlusselement (9) ein Band variabler Länge ist, das die natürliche Leitung (10) umschließt,
- die Betätigungsvorrichtung (2) ein mechanisches Übertragungselement (11) umfasst, welches das Band und einen Aktuator verbindet, der ausgebildet ist, um den Lauf des mechanischen Übertragungselements einzustellen,
wobei die Variation der von dem Band auf die Leitung (10) ausgeübten Kompression durch Einstellung des Laufs des mechanischen Übertragungselements durchgeführt wird.

12. System nach Anspruch 11, wobei der Sensor ausgebildet ist, um die mechanische Spannung des mechanischen Übertragungselements zu messen und die Verarbeitungseinheit konfiguriert ist, um aus der gemessenen mechanischen Spannung die von dem Band auf die natürliche Leitung (10) ausgeübte Kompression abzuleiten.

13. System nach einem der Ansprüche 11 oder 12, wobei der repräsentative Parameter der Lauf des mechanischen Übertragungselements zwischen dem Aktuator und dem Verschlussband ist, um eine bestimmte Kompression der Leitung (10) zu erreichen.

14. System nach einem der Ansprüche 11 bis 13, wobei die Verarbeitungseinheit einen Speicher umfasst, in welchem ein Verhältnis zwischen dem Lauf des mechanischen Übertragungselements und der mechanischen Spannung des Übertragungselements oder der von dem Band ausgeübten Kompression gespeichert ist.

15. System nach einem der Ansprüche 1 bis 14, **dadurch gekennzeichnet, dass** der repräsentative Parameter für eine bestimmte Bedingung des Patienten definiert ist und das Verschlusssystem einen Sensor umfasst, der ausgebildet ist, um die bestimmte Bedingung des Patienten zu ermitteln.

16. System nach einem der Ansprüche 1 bis 15, wobei die Verarbeitungseinheit in der Steuereinheit (7) des Verschlusssystems eingeschlossen ist.

17. System nach einem der Ansprüche 1 bis 16, umfassend ferner Sendemittel eines Alarms an einen Benutzer, wenn das Kriterium der Detektion einer Atrophie der natürlichen Leitung erfüllt ist.

18. Verschlusssystem nach einem der Ansprüche 1 bis 17, bestehend aus einem künstlichen Harnschließmuskel.

## Claims

1. An occlusive system implantable in an animal or human body for occluding a natural conduit (10) of an animal or human body, comprising:
- an occlusive element (9) designed to enclose part of the natural conduit (10) to be occluded,
- an activation device (2) of said occlusive element (10) for varying the compression exerted by said occlusive element (9) on said conduit (10),
- a control unit (7) adapted to stress the activation device (2) so as to exert determined compression on the conduit (10), and
- a detection device of atrophy of said natural conduit (10), comprising:
(i) a sensor adapted to measure the compression exerted by the occlusive element (9) on the natural conduit (10), and
(ii) a processing unit adapted to:
• determine a representative parameter of stress to be applied to the activation device (2) to obtain a determined compression of the conduit (10) by the occlusive element (9),
• monitor said parameter over time, and
• detect atrophy of the natural conduit (10) when said representative parameter fulfils a predetermined criterion.

2. The system according to claim 1, wherein said predetermined criterion is selected from one of the following conditions or a combination of said conditions:
- the representative parameter is greater than a fixed value,
- the representative parameter is greater than a percentage of a value of said representative parameter measured initially,
- a value coming from a mathematical function constructed from a database of values of said representative parameter measured periodically over time is greater than a determined value.

3. The system according to one of claims 1 or 2, said system being an occlusive hydraulic system wherein:
- the occlusive element (9) is an inflatable cuff comprising a variable volume of fluid,
- the activation device (2) comprises a tank (4) containing fluid, in fluidic connection with said inflatable cuff,
the variation in compression exerted by the cuff on the conduit (10) being done by transfer of an adjustable volume of said fluid between the tank (4) and the cuff.

4. The system according to claim 3, wherein the sensor is adapted to measure the compression of the natural conduit directly between the occlusive element (9) and the natural conduit (10).

5. The system according to claim 3, wherein the sensor is adapted to measure the pressure in a region of said hydraulic circuit and the processing unit is configured to deduce from said measurement the compression exerted by the cuff on the natural conduit (10).

6. The system according to one of claims 3 to 5, wherein said representative parameter is the volume of fluid to be transferred from the tank (4) to the inflatable cuff to achieve determined compression of the conduit (10).

7. The system according to claims 3 to 6, wherein the processing unit comprises a memory wherein a relation between the pressure in the hydraulic circuit and the volume transferred to the inflatable cuff is recorded.

8. The system according to one of claims 3 to 7, wherein the tank (4) is a tank of variable volume comprising a mobile wall and wherein the processing unit is configured to determine the volume of fluid transferred from measurement of the displacement of said mobile wall.

9. The system according to one of claims 3 to 8, further comprising means for detecting a slow leak in the hydraulic circuit, said means being configured for:
- measuring the evolution of the pressure in the hydraulic circuit for a determined stress of the activation device (2),
- detection of a slow leak in the hydraulic circuit when the pressure measured in said circuit for said determined stress of the activation device (2) fulfils a predetermined criterion.

10. The system according to claim 9, **characterized in that** said predetermined criterion for detecting a slow leak is selected from one of the following conditions or a combination of said conditions:
- the pressure in the hydraulic circuit is less than a fixed value,
- the pressure in the hydraulic circuit is less than a percentage of a value of the pressure measured initially for said determined stress of the activation device, and
- a value coming from a mathematical function constructed from a database of values of said pressure for said stress of the activation device, said pressure being recorded periodically over time, is less than a determined value.

11. The system according to one of claims 1 or 2, said system being a mechanical occlusive system wherein:
- the occlusive element (9) is a band of variable length enclosing the natural conduit (10),
- the activation device (2) comprises a mechanical transmission element (11) connecting said band and an actuator adapted to adjust the course of said mechanical transmission element,
the variation of the compression exerted by the band on the conduit (10) being done by adjustment of the course of said mechanical transmission element.

12. The system according to claim 11, wherein the sensor is adapted to measure the mechanical tension of said mechanical transmission element and wherein the processing unit is configured to deduce from said measured mechanical tension the compression exerted by the band on the natural conduit (10).

13. The system according to one of claims 11 or 12, wherein said representative parameter is the course of said mechanical transmission element between the actuator and the occlusive band to reach determined compression of the conduit (10).

14. The system according to one of claims 11 to 13, wherein the processing unit comprises a memory wherein a relation between the course of the mechanical transmission element and the mechanical tension of said transmission element or the compression exerted by the band is recorded.

15. The system according to one of claims 1 to 14, **characterized in that** said representative parameter is defined for a determined condition of the patient and **in that** said occlusive system comprises a sensor adapted to detect said determined condition of the patient.

16. The system according to one of claims 1 to 15, wherein the processing unit is included in the control unit (7) of the occlusive system.

17. The system according to one of claims 1 to 16, further comprising means for sending an alarm to a user if the detection criterion of atrophy of the natural conduit is fulfilled.

18. The occlusive system according to one of claims 1 to 17, consisting of an artificial urinary sphincter.
